(19) Europäisches Patentamt
European Patent Office
Office européen des brevets

(11) **EP 3 524 953 A1**

(12) **EUROPEAN PATENT APPLICATION**

(43) Date of publication:
**14.08.2019 Bulletin 2019/33**

(51) Int Cl.:
**G01L 11/02** $^{(2006.01)}$

(21) Application number: **18155462.7**

(22) Date of filing: **07.02.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(71) Applicant: **Koninklijke Philips N.V.
5656 AE Eindhoven (NL)**

(72) Inventors:
• **MUELLER, Manfred
5656 AE Eindhoven (NL)**
• **SCHLEIPEN, Johannes Joseph Hubertina
Barbara
5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **DISTRIBUTED INTRAVASCULAR FIBER BRAGG PRESSURE SENSOR**

(57) The present invention relates to a pressure sensing device (10) comprising an optical fiber (12), the optical fiber (12) comprises a central axis (L) and at least one optical fiber core (14), the at least one optical fiber core (14) having one or more reflective FBG structures, and a coating (16) surrounding the optical fiber (12), the coating (16) having mechanical properties which are radially asymmetric along the central axis (L).

FIG.3

EP 3 524 953 A1

**Description**

FIELD OF THE INVENTION

The present invention relates to pressure sensing. In particular, the present invention discloses a pressure sensing device wherein a coating surrounds an optical fiber with one or more reflective Fiber-Bragg-Grating structures. The coating exhibits mechanical properties which are radially asymmetric along the central axis of the device. The present invention furthermore discloses a corresponding method for determining a pressure.

BACKGROUND OF THE INVENTION

A significant technical field for pressure sensing is intravascular pressure sensing requiring high sensitivity and accuracy. Intravascular pressure measurements, such as guidewires with a single integrated pressure sensor, are an important tool to measure the patency and the functioning of blood vessels. The problem of these single sensor guidewires resides in that the physician has to move the guidewire or the catheter each time he/she wants to measure pressure at a different location along the blood vessel. This results in "losing wire position" which necessitates time-consuming repositioning of the wire and may even lead to pressure sensing at the wrong location inside the blood vessel. Moving the guidewire back and forth along the arteries does not only lengthen the procedure, but also carries the risk of adverse events, such as vessel dissection.

To solve this issue one approach resides in the provision of guidewires or catheters with multiple sensors that can measure pressures at various points along the length of the guidewire or catheter. Unfortunately, simply adding multiple additional (capacitive or piezoelectric) pressure sensors to the guidewire causes multiple problems: the additional sensors and their cabling requirements make the resulting guidewire too stiff, too fragile, and too expensive. One possible solution resides in choosing a different sensor technology, one that is more suited to multiple or distributed sensors.

Fiber-Bragg Gratings (FBGs) have been suggested as one approach for distributed sensing. FBGs are optical fibers with a periodic variation of the refractive index. Light will be reflected by a FBG only when its wavelength $\lambda$ fulfils the Bragg condition:

$$\text{Light will be reflected by a FBG only when its wavelength } \lambda \text{ fulfils the Bragg condition: } \lambda = 2\,\Gamma n_{eff} \qquad (1),$$

wherein $\Gamma$ and $n_{eff}$ are the grating period of the grating and the effective refractive index of the fiber core, respectively (https://en.wikipedia.org/wiki/Fiber_Bragg_grating). Light that does not fulfil the Bragg condition will pass through the FBG. FBGs are suited to distributed sensing, because multiple Bragg gratings can be integrated in a single optical fiber and can be read out individually. This is used for example in optical shape sensing technology, where optical fibers with integrated FBGs can detect bending of the fiber over a distance of several meters. FBGs will sense any change of the period length r or the refractive index n of the grating. Such a change can be induced by e.g. temperature, strain or pressure.

The problem with using FBGs as blood pressure sensors is their low sensitivity to pressure and their comparatively high sensitivity to temperature and strain. The pressure range that is required for blood pressure measurements is from about -30 mmHg to 300 mmHg (approximately -4 kPa to 40 kPa). Precision should be $\leq$ 2 mmHg (i.e. $\leq$ approx. 0.3 kPa).

Silica FBGs have a pressure sensitivity of approximately 0.003 pm/k Pa (0.0004 pm/mmHg) but a temperature sensitivity of about 10 pm/°C and a strain sensitivity of about $\approx$1.3 pm/p$\varepsilon$, where 1 picostrain p$\varepsilon$ is defined as $10^{-12}$ times the relative physical length change $\Delta L/L$ (K. Bhowmik, et al., "Experimental Study and Analysis of Hydrostatic Pressure Sensitivity of Polymer Fibre Bragg Gratings", J. Lightwave Tech. 33(12), 2456-2462 (June 2015); US 2015/0141854 A). A pressure change of about 2 mmHg will therefore cause a wavelength shift in the FBG of about 0.0008 pm. This is a very small wavelength shift. For comparison typical commercially available FBG interrogators have a wavelength resolution of about 1 pm (see http://www.micronoptics.com, http://www.opto-works.co.jp/FBG/BaySpec-Datasheet-FBGA-IRS-R(1).pdf, or https://www.hbm.com/en/4604/fs22-industrial-braggmeter-optical-interrogator/) with some reaching resolutions as low as 0.05 pm (see http://www.technobis.com).

The reason for the low pressure sensitivity of typical FBG is the low elasticity of silicon fibers that form the basis of commercially available FBGs. Hydrostatic pressure changes the physical length L of a fiber leading to a change in r. In addition, pressure changes the refractive index $n_{eff}$ of the fiber. A hydrostatic pressure change of $\Delta P$ will cause a change $\Delta \lambda$ in the Bragg wavelength according to M. G. Xu et al., "Optical Fibre Sensor for High Pressure Measurement Using an in-fibre Grating", http://eprints.soton.ac.uk/77273/1/663.pdf (1993):

$$\frac{\Delta\lambda}{\lambda} = \left(-\frac{(1-2\nu)}{E} + \frac{n_{eff}^2}{2E}(1 - 2\nu)(2p_{12} + p_{11})\right)\Delta P \qquad (2),$$

wherein E is the Young's modulus, $\nu$ the Poisson ratio, $p_{12}$ and $p_{11}$ are components of the strain optic tensor. Typical values for glass are E=70 GPa and $\nu$ = 0.17, $p_{12}$=0.27 and $p_{11}$ = 0.17. It's obvious from this equation that the wavelength shift is inversely proportional to E.

It is possible to enhance the pressure sensitivity by manufacturing FBGs from materials with a lower Young's modulus. K. Bhowmik, et al., "Experimental Study and Analysis of Hydrostatic Pressure Sensitivity of Polymer Fibre Bragg Gratings", J. Lightwave Tech. 33(12), 2456-2462 (June 2015) discloses the manufacture of FBGs in self-made polymer fibers with a wavelength sensitivity of $\Delta\lambda/\Delta P \approx 0.027$ pm/mmHg in the original fibers and up to $\Delta\lambda/\Delta P \approx 0.1$ pm/mmHg after etching the fibers to a diameter of 55 $\mu$m. Such polymer FBGs are, however, not suitable for use in commercial applications at the moment. There are no polymer FBGs commercially available. Single-mode polymer fibers are a small niche. These fibers and the required support techniques (splicing, cleaving, polishing, FBG writing, etc.) are not available. This is partly due to well-known issues with polymer fibers, such as plastic deformation, lack of homogeneity, change in properties due to water absorption, etc. Last but not least using polymer FBGs will increase strain and temperature sensitivity as well, requiring strain and temperature compensation techniques.

A number of alternative ways to increase the pressure sensitivity of FBGs have been proposed. V.R. Pachava, et al, "A high sensitive FBG pressure sensor using thin metal diaphragm" discloses a pressure sensitivity of $\Delta\lambda/\Delta P > 4$ pm/mmHg by integrating the FBG into a membrane. The pressure-induced bending of the membrane translates axial hydrostatic pressure into a shape change (and therefore a change in longitudinal strain) to which FBGs are much more sensitive. This principle is, however, not suited for miniaturization or distributed sensing.

Many other approaches have been proposed to increase the pressure sensitivity of FBGs, including the use of holey fibers, and the use of fiber Fabry-Pérot-resonators. For instance, US 5,297,437 A discloses a fiber Fabry-Pérot-interferometer embedded in an elastic material.

No approach so far has resulted in a design that is suitable for distributed, high sensitive pressure determination, such as intravascular blood pressure measurements.

## SUMMARY OF THE INVENTION

It is an object of the present invention to provide a pressure sensing device based on optical fibers and a method determining pressure, wherein the above mentioned drawbacks have been overcome. In particular, an objective resides in the provision of pressure sensing devices based on optical fibers, wherein the optical fibers exhibit a sufficient sensitivity to low pressures, such as the hydrostatic pressure prevailing in blood vessels. A further objective resides in the provision of a distributed sensing device. Another objective resides in the provision of pressure sensing devices based on optical fibers with decreased or eliminated temperature sensitivity.

In a first aspect of the present invention a pressure sensing device is presented that comprises:

an optical fiber comprising or consisting of a central axis and at least one optical fiber core, the at least one optical fiber core having one or more reflective FBG structures, and
a coating surrounding the optical fiber, the coating having mechanical properties which are radially asymmetric along the central axis.

In a further aspect of the present invention method for determining a pressure is presented. The method comprises
optically determining bending of an optical fiber, the optical fiber comprising or consisting of a central axis and at least one optical fiber core, the at least one optical fiber core having one or more reflective FBG structures, wherein a coating surrounds the optical fiber, the coating having mechanical properties which are radially asymmetric along the central axis, and
calculating the pressure or a pressure difference from the bending of the coating.

In yet further aspects of the present invention, there are provided a system comprising the present pressure sensing device, and a computer program which comprises program code means for causing a computer to perform the steps of the method disclosed herein when said computer program is carried out on a system comprising the present pressure sensing device.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

The present invention is based on the finding that pressure sensitivity of optical fibers may be improved while at the same time decreasing their temperature sensitivity, by integrating one or more reflective FBG structures into a device whose mechanical properties are radially asymmetric along the central axis of the optical fiber.

Hence, the mechanical properties, in particular one or more of the Young's modulus of elasticity, the thermal expansion coefficient, and the Poisson ratio, of the coating are not radially symmetric on all points along the length of the device but vary with the azimuthal angle φ. Any pressure, in particular hydrostatic pressure, will exert radial forces on the device and particularly the one or more reflective FBG structures. The radial forces can lead to a local radial deformation or bending of the device and/or the optical fiber. Deformation or bending can be detected with existing technologies, in particular with optical shape sensing technology, such as FORS technology by Philips. This local bending of the device results in a larger strain signal, and hence pressure sensitivity, as compared to plain longitudinal compression/expansion of the device.

The present invention thereby provides a new way to enhance the sensitivity of an optical fiber comprising or consisting of a central axis and at least one optical fiber core, the at least one optical fiber core having one or more reflective FBG structures, in particular FBGs, to pressure, in particular hydrostatic pressure. The present invention assists in avoiding most of the limitations of prior art approaches and is suitable for distributed sensing in e.g. a guidewire-sized device. It is possible to optimize the design in such a way that it is only sensitive to pressure, such as hydrostatic pressure, but not to temperature.

As already indicated above, the present sensing principle can be beneficially combined with optical shape sensing technologies, in particular FORS technology by Philips. For example, it could add pressure sensitivity to a shape-sensing guidewire. With FORS using an optical shape sensing fiber integrated in an elongated device, the three-dimensional shape of the device can be known and thus be made "visible" up to the tip of the device, although the device itself may be invisible for the user's eyes. In medical applications, FORS fibers can be integrated into a wide range of elongated medical devices like catheters, guidewires or endoscopes to provide live guidance or navigation of medical procedures. It is to be understood that the present invention is not limited to medical applications, but can be also widely used in industrial fields of technology. With FORS, two or more elongated devices can be tracked simultaneously. In this case, each of the tracked devices is equipped with an FORS fiber. The optical fibers of the individual devices are interrogated simultaneously, and the 3D shape of each of the devices can be reconstructed, accordingly. The reconstructed shapes can be visualized together on a display or screen of a monitor.

In general, the present pressure sensing device is suitable for distributed sensing/determining of low pressures of about -8 kPa to 80 kPa with a high precision of $\leq$ 10 mmHg. In particular, the present pressure sensing device is applicable to intravascular blood pressure sensing, in particular guidewires for intravascular blood pressure sensing. Intravascular blood pressure sensing is relevant for interventional cardiology, such as Fractional Flow Reserve (FFR), but also for applications in the peripheral vasculature, and potentially for some kinds of structural heart interventions and for predicting heart failure.

The "central axis" or "neutral axis" of the optical fiber may correspond to the longitudinal axis of a cylindrical coordinate system. In case the optical fiber is a multicore optical fiber, the central axis preferably corresponds to the axis of one of the at least one optical fiber cores. Alternatively, the central axis is offset of and parallel to the axis of one of the at least one optical fiber cores.

The optical fiber may be a single mode or multimode optical fiber.

The optical fiber may comprise any number of optical fiber cores, such as 1, 2, 3, 4, 5, 6, 7, 8, 9, 10 or even more than 15. A multicore optical fiber, preferably a multicore FBG fiber, is like the one used in optical shape sensing (preferably with one extra core because hydrostatic pressure adds one extra degree of freedom). This way the guidewire can sense both its position and the surrounding hydrostatic pressure.

Preferably, the optical fiber is a glass fiber, more preferably a silica fiber. The use of these optical fibers and/or optical fiber cores made of these materials has the advantage that suitable techniques, e.g. for preparing and/or modifying the optical fibers and/or optical fiber cores, are already available and well known in the art.

The expression "one or more reflective FBG structures" as used herein pertains to one or more periodical or quasi-periodical structures, such as chirped structures, embedded into the optical fiber core. These one of more fiber Bragg gratings (FBGs) structures enable Bragg reflection. A fiber optic Bragg grating is a short segment of optical fiber that reflects particular wavelengths of light and transmits all others. This is achieved by adding a periodic variation of the refractive index in the fiber core, which generates a wavelength-specific dielectric mirror. A fiber Bragg grating can therefore be used as an inline optical filter to block certain wavelengths, or as a wavelength-specific reflector. A fundamental principle behind the operation of a fiber Bragg grating is Fresnel reflection at each of the interfaces where the refractive index is changing. For some wavelengths, the reflected light of the various periods is in phase so that constructive interference exists for reflection and, consequently, destructive interference for transmission. The Bragg wavelength is sensitive to strain as well as to temperature. This means that Bragg gratings can be used as sensing elements in fiber optical sensors. Preferably, the optical fiber core has at least one long or multiple short FBGs written in. A long FBG pertains to a long-period fiber grating having a period which is much greater, such as 1000 times greater or more, than the wavelength of radiation propagating in the fiber. The fiber may contain one or more optical fiber cores with their own FBGs. If a single optical fiber core is used, the core is preferably offset from the neutral axis.

The at least one optical fiber core has one or more reflective FBG structures. Preferably, each optical fiber core has at least one or more reflective FBG structures such as 2, 3, 4, 5, 6, 7, 8, 9, 10 or even more than 15.

The optical fiber core may comprise, in addition to the one or more FBG reflective structures, non-periodic structures giving rise to random variations of the refractive index, e.g. by Rayleigh scattering. Rayleigh scatter maybe used in standard single-mode communications fiber enabling exploiting the inherent backscatter in conventional optical fiber. Rayleigh scatter occurs as a result of random fluctuations of the index of refraction in the fiber core. These random fluctuations can be modelled as a Bragg grating with a random variation of amplitude and phase along the grating length. By using this effect in three or more cores running within a single length of multi-core fiber, the 3D shape and dynamics of the surface of interest can be followed.

The coating may be produced in different manners. For instance, a conventional optical fiber having at least one fiber core may be provided. Surface portions of the at least one fiber core are exposed by using abrasives, preferably a laser. Alternatively, chemical or mechanical abrasives may be employed. The surface portions are than coated with a material of mechanical properties which are radially asymmetric along the central axis. A particular advantage resides in that the materials, such as polymers, preferably rubbers, having the required mechanical properties are known in the art. The same applies to the required coating techniques.

It is preferred that the coating has a constant thickness along the axis L. In said case the mechanical properties arise from the use of different coating materials.

Since optical fibers, such as guidewires and microcatheters, are of elongated cylindrical shape, cylindrical coordinates may be employed. The axial dimension is the length L, which goes from proximal to distal. Forces, stress, strain and other vectors that are (approximately) parallel to the length are called longitudinal. Forces and other vectors that are (approximately) perpendicular to the axis are called radial. Properties and forces can vary with the azimuth angle $\varphi$. Properties that are independent of the azimuth $\varphi$ are called radial symmetric. Hydrostatic pressure is an example of a radial force that is also radial symmetric.

The expression "Poisson ratio" as used herein is a measure of the Poisson effect and preferably is the signed ratio of transverse strain to axial strain as defined in https://en.wikipedia.org/wiki/Poisson%27s_ratio.

According to one embodiment of the present invention, the pressure sensing device is adapted to determine a local pressure, the local pressure exerting radial forces on the coating.

The pressure may be any kind of pressure. The pressure is preferably a local hydrostatic pressure, such as a local hydrostatic pressure prevailing in a blood vessel. At least portions of the material of the coating are adapted to deform or bend under the pressures to be detected. Preferably, the pressures to be detected are in the range of from -8 kPa to 80 kPa, preferably -4 kPa to 40 kPa, or -2 kPa to 20 kPa. Alternatively or in addition, the precision is $\leq$ 10 mmHg, $\leq$ 5 mmHg, $\leq$ 3.5 mmHg, or preferably $\leq$ 2 mmHg, such as $\leq$ 1.5 mmHg, $\leq$ 1 mmHg, or $\leq$ 0.5 mmHg.

According to another embodiment of the present invention, the mechanical properties are balanced such that thermal influences and/or strain influences are minimized so that any deformation/bending of the optical fiber will be preliminarily caused by pressure. The wording "preliminarily caused by pressure" preferably has the meaning that at least 90%, such at least 95%, at least 98%, at least 99%, or at least 99,5% of the bending are caused by pressure. This may be calculated and/or experimentally determined. Preferably, only a pressure change results in a deformation of the optical fiber.

Hence, the coating material does not deform/bend or deforms/bends to a minimal extent upon exertion of other environmental effects, preferably thermal effects and/or strain. This may be achieved e.g. by using materials with one or more of similar thermal expansion coefficients, similar Young's modulus of elasticity, and different Poisson ratio. Preferably, the materials exhibit at least similar thermal expansion coefficients for reducing thermal effects or even completely cancelling them. These material properties are known and may be derived from common textbooks. It will be appreciated that the absolute value of these material properties is not of importance since merely their relationship with respect to each other needs to be considered. Device and method for measuring the material properties in question are not decisive but only the possibility that the values may be compared with each other. This can be established by using any device but preferably using the same device and same method for materials to be compared.

The balanced mechanical properties have the effect that the present device does not bend in a specific direction upon exerting a pressure on the device but rather that the mechanical properties of the coating are altered such that different bending directions are cancelling out each other. As a result the present device may maintain an essentially linear shape even under sensing conditions of a plurality different local pressures. In other words, the optical fiber is subjected to local bending which may be measured but which does not influence the overall direction of the optical fiber.

An essential linear shape preferably means a shape wherein a first end of the optical fiber forms the tip and the second end of the optical fiber any point of the circle area of a(n imaginary) right circular cone. Any point between the first end and the second end of the optical fiber is located within the cone's volume or on the cone's surface. The height of the cone is preferably about 200 $\mu$m to 800 $\mu$m, such as 300 $\mu$m to 700 $\mu$m, 400 $\mu$m to 600 $\mu$m, or 550 $\mu$m to 650 $\mu$m, with

a radius of about 35 μm to 45 μm, such as 38 to 42 μm, or 40 μm.

Other effects acting on the coating, such as strain pressure and/or temperature, can also be partially or completely compensated by using optical shape sensing algorithms. In particular, the already known optical shape sensing technologies may be easily applied. It is also conceivable to employ a machine-learning algorithm based on a comparison of a system subjected to different variations of local pressure, temperature and strain and teaching the system such that the later two are effectively cancelled out.

Deriving an exact value for the bending effect caused by hydrostatic pressure for the device is preferably performed using numerical simulations. To derive an approximate value, equations may be adapted that have been developed to derive the bending radius of bimetallic strips caused by thermal expansion. Linear thermal expansion is given by (https://en.wikipedia.org/wiki/Thermal_expansion#Coefficient_of_thermal_expansion):

$$\frac{\Delta l}{l} = \alpha \Delta T \qquad (3),$$

wherein $\alpha$ is the coefficient of thermal expansion and $\Delta T$ is the change in temperature. In comparison, the longitudinal change in length due to pressure can be approximated by:

$$\frac{\Delta l}{l} \approx \frac{\nu}{E} \Delta P \qquad (4).$$

It's therefore possible to adapt the Timoshenko equations for a bimetallic beam (see e.g. https://en.wikipedia.org/wiki/Bimetallic_strip) by replacing $\alpha$ with $\dfrac{\nu}{E}$ and replacing $\Delta T$ with $\Delta P$. The curvature $\kappa$ of the bent device can then be estimated by:

$$\kappa = \frac{12\,E_1 E_2 \left(\frac{\nu_2}{E_2} - \frac{\nu_1}{E_1}\right)}{h\,(E1^2 + 14 E_1 E_2 + E_2^2)}\,\Delta P, \qquad (5),$$

wherein h is the thickness of the coating. This simple estimation makes some strong, simplifications: it derives from a quadratic rather than a round cross section and it ignores the influence of the optical fiber.

According to a further embodiment of the present invention, the coating has at least two longitudinal sections, each of the at least two longitudinal sections having a first annular subsection made of a first material and a second annular subsection made of a second material being different from the first material, wherein the first material has a thermal expansion coefficient $CTE_1$, a Young's modulus of elasticity $E_1$, and a Poisson ratio $\nu_1$ and the second material has a thermal expansion coefficient $CTE_2$, a Young's modulus of elasticity $E_2$, and a Poisson ratio $\nu_2$, wherein at least one of a deviation of $CTE_1$ and $CTE_2$ is $\leq$ 10 % referred to the lowest value of $CTE_1$ and $CTE_2$, a deviation of $E_1$ and $E_2$ is $\leq$ 10 % referred to the lowest value of $E_1$ and $E_2$, and a deviation of $\nu_1$ and $\nu_2$ is $\geq$ 75 % referred to the lowest value of $\nu_1$ and $\nu_2$. Instead of the deviation of $\nu_1$ and $\nu_2$, the difference between absolute values for $\nu_1$ and $\nu_2$ maybe considered. This difference is 0.05 or more, such as 0.075 or more, 0.1 or more, 0.15 or more, 0.2 or more, or 0.25 or more. In said case, $\nu_1$ and $\nu_2$ are determined using the same conditions/measurement methods or, preferably derived from the same source of literature provided that there is no indication about using different conditions/measurement methods for determining $\nu_1$ and $\nu_2$. A suitable source of literature for some values is for instance https://en.wikipedia.org/wiki/Poisson%27s_ratio.

Respective values for such similar thermal expansion coefficients, a similar Young's modulus of elasticity, and different Poisson ratio may be derived from available text books. Alternatively, these values may be measured by using the any device and method with the proviso of using the same device and method for materials to be compared. The values employed for the thermal expansion coefficients, Young's modulus of elasticity, and Poisson ratio, respectively, exhibit an error margin which is less than 50 % of the deviation referred to the lowest value. In case, however, the error margin is exceeds said range, the arithmetic average of the error margin may be employed. It will be appreciated that not all three conditions need to be fulfilled at the same time. Rather each combination is possible, such as similar thermal expansion coefficients and different Poisson ratios, or similar Young's modulus of elasticity and different Poisson ratios.

It is particular preferred that $\nu \approx 0.49$, i.e. $\nu_1$ is 0.46 to 0.5, such as 0.48 to 0.49, or 0.49 and the second coating material has Poisson's number $\nu_2$ of about 0.2 or less, such as 0.18 or less, or 0.15 or less.

Preferably, the deviation of $CTE_1$ and $CTE_2$ is $\leq$ 8 %, such as $\leq$ 5 %, or $\leq$ 2 %. Still preferably, the deviation of

$E_1$ and $E_2$ is $\leq 8\%$, such as $\leq 5\%$, or $\leq 2\%$. Still preferably, the deviation of $\nu_1$ and $\nu_2$ is $\geq 100\%$, such as $\geq 110\%$, or $\geq 120\%$.

According to an embodiment of the present invention, wherein each of the at least two longitudinal sections has a first annular subsection and a second annular subsection, the first annular subsection having an azimuth $\varphi_1$, wherein $0° \leq \varphi_1 < 180°$, the second annular subsection having an azimuth $\varphi_2$, wherein $\varphi_1 + \varphi_2 = 360°$. The first annular subsection may have any azimuth $\varphi_1$ in the above mentioned range, such as 0° to 45°, 0° to 60°, 0° to 90°, 0° to 120°.

Preferably, the central axis corresponds to the longitudinal axis of a cylindrical coordinate system. This holds particularly true if an optical fiber with a single optical fiber core is considered. In case the optical fiber has two or more fiber cores, the central axis preferably corresponds to the axis of one of the at least one optical fiber cores or with the geometric center of the cross section of the fiber. Alternatively, the central axis is offset of and parallel to the axis of one of the at least one optical fiber cores.

Any number of longitudinal sections, such as 2, 3, 4, 5, 6, 7, 8, 9, 10, 20, 30, 40, 50, or 100 maybe employed. Each longitudinal section is made of at least two annular subsections. The two annular subsections have different mechanical properties, in particular at least one or more of different thermal expansion coefficients, Young's modulus of elasticity, and Poisson ratio in the ranges as indicated above.

Preferably only two annular subsections are employed. It is particularly preferred that the first annular subsection has an annular azimuth $\varphi_1$, wherein $0° \leq \varphi_1 < 180°$, and the second annular subsection has an annular azimuth $\varphi_2$, wherein $180° \leq \varphi_2 < 360°$. Alternatively, the first annular subsection has an annular azimuth $\varphi_1$ and the second annular subsection has an annular azimuth $\varphi_2$. Any of $\varphi_1$ and $\varphi_2$ is from 100° to 200°, in particular 120° or 180°, and $\varphi_1 + \varphi_2 = 360°$.

According to another embodiment of the present invention, first annular subsections of the at least two longitudinal sections are staggered around the optical fiber such that the first annular subsections of the at least two longitudinal sections do not overlap.

For example, the first annular subsection of a first section encompasses an angle of $0° \leq \varphi < 180°$ and the first annular subsection of a further first longitudinal section is $180° \leq \varphi < 360°$. It is preferred that a ratio of annular subsections with different angle $\varphi$ is 0.8 to 1.2, such as 0.9 to 1.1, preferably 0.95 to 1.05, such as 1.0. The first annular subsections may be adjacent to each other or shared by an axially symmetric coating. The axially symmetric coating is a longitudinal section without distinguished mechanical properties, such as a longitudinal section which is completely made of a specific material.

It is furthermore conceivable that the azimuthal starting positions of the first annular subsections and/or the second annular subsections are a multiple of $\pi$ resulting in an in-plane oscillatory behavior of the optical fiber in response to a pressure. Alternatively, the azimuthal starting positions of the first annular subsections and/or the second annular subsections are at an azimuthal angle different than $\pi$ resulting essentially in the shape of a helix of the optical fiber in response to a pressure. This results in a higher sensitivity of the present device to e.g. pressure changes. Still alternatively, the azimuthal starting position of the first annular subsections and/or the second annular subsections changes continuously as a function of longitudinal position along the fiber.

According to still another embodiment of the present invention, each of the longitudinal sections encompasses at least one of the one or more reflective FBG structures.

Any number of one or more reflective FBG structures, particularly FBGs, may be encompassed within the optical fiber core(s) of a longitudinal section, such as 1 or more, 2 or more, 3 or more, 4 or more, 5 or more, or 10 or more. The at least one of the one or more reflective FBG structures is/are preferably located symmetric, such as centrosymmetric, within the optical fiber core(s) of the respective longitudinal sections. A higher number of the one or more reflective FBG structures improves sensitivity of pressure measurement and may also assist in a higher accuracy of the measurements.

According to a further embodiment of the present invention, the present pressure sensing device further comprises at least one axially symmetric coating with $0° \leq \varphi < 360°$.

The axially symmetric coating is a longitudinal section without distinguished mechanical properties, such as a longitudinal section which is completely made of a specific material.

The axially symmetric coating is usually made of the same material. This material forms a complete annulus around the optical fiber. Any number of areas covered with the axially symmetric coating may be present, such as at least 1 or more, such as 2, 3, 4, 5, 6, 7, 8, 9, or 10. Areas covered with the axially symmetric coating may by sandwiched by longitudinal sections. It is preferred that ratio of number of areas covered with the axially symmetric coating and longitudinal sections is 0.8 to 1.2, such as 0.9 to 1.1, or 1.0.

According to an embodiment of the present invention, the first annular subsection has $0° \leq \varphi < 180°$, and the second annular subsection has $180° \leq \varphi < 360$.

According to another embodiment of the present invention, a system for pressure sensing is provided. The system comprises the present pressure sensing device, and an interventional device, wherein one end of the optical fiber is coupled to the interventional device.

The interventional device is preferably an intravascular device. The interventional device may be a catheter, a guidewire or the like suitable for being introduced into a blood vessel of a patient. The interventional device may comprise an elongated shaft of any suitable length, of e.g. 1 m. The system may also comprise a workstation or console, to which the interventional device may be connected for communication, in particular optical communication of one or more console components, such as an optical interrogator and/or a console component adapted for evaluation of the signals. A portion of the interventional device comprising the present pressure sensing device may be configured to be insertable into a blood vessel. The interventional device may have in addition to the present pressure sensing device any number of additional (conventional) optical fibers adapted for different purposes, such as optical shape sensing (OSS) as such. Optical shape sensing is an optical measurement technique for determining the position and shape of a structure in a three dimensional space thereby affording an exact correlation of e.g. blood pressure with respect to specific body areas of a patient. It is preferred that the system also provides a display or the like simultaneously showing both hydrostatic pressure and device position to the user. It will be readily understood that the present invention is not limited to an interventional device, rather any device or system for determining pressure may be encompassed.

According to still another embodiment of the present invention, the interventional device is a guidewire or a catheter.

According to a further embodiment of the present invention, the interventional device comprises an optical interrogator.

The optical interrogator is for generating measurement signals being scattering spectrum signals, in particular reflection spectrum signals, indicative of an amplitude and/or a phase of a scattering in an elongated portion of the shape sensing element. Local strain data reflecting the local pressure, in particular the local hydrostatic pressure, acting on different longitudinal portions of the optical fiber can be derived from the scattering spectrum signals, wherein local curvature and/or torsion angle of the optical fiber can be obtained from the local strain data. The optical interrogator maybe furthermore adapted for generating respective measurement signals of any additional (conventional) optical fiber(s) adapted for different purposes.

According to a preferred embodiment of the present invention, the pressure is a blood pressure in a blood vessel of a patient.

According to another preferred embodiment of the present invention, calculating the pressure difference from the bending of the optical fiber is performed by calibration measurements and/or FEM (Finite element method) simulations.

Calibration measurements and FEM simulations are well known in the art. Alternatively or in addition, the following equation may be employed:

$$\kappa = \frac{12\,E_1 E_2 \left(\frac{v_2}{E_2} - \frac{v_1}{E_1}\right)}{h\,(E1^2 + 14 E_1 E_2 + E_2^2)}\,\Delta P, \tag{5}$$

wherein $\kappa$ is the curvature of the bending of the optical fiber (12), $E_1$ is the Young's modulus of elasticity of a first material of the coating (16), $E_2$ is the Young's modulus of elasticity of a second material of the coating (16), $v_1$ is the Poisson ratio of the first material of the coating (16), $v_2$ is the Poisson ratio of the second material of the coating (16), h is the thickness of the coating (16) and $\Delta P$ is the pressure difference.

Preferably, a method for preparing a pressure sensing device may be provided. The method comprises providing an optical fiber comprising a central axis L and at least one optical fiber core, the at least one optical fiber core having one or more reflective FBG structures, coating the optical fiber such that the coating surrounds the optical fiber and that the coating has mechanical properties which are radially asymmetric along the central axis.

Preferably, coating the optical fiber with a coating comprises applying a coating material on the optical fiber in a radial asymmetric manner.

Preferably, the method further comprises chemically and/or physically treating the coating.

Chemically treating encompasses for instance etching of surface portions. Physically treating may encompass radiation of a polymer of a coating with a suitable light source for hardening the same and/or etching of surface portions by using a laser. It is preferred treating the same material with different light sources and/or light intensities for generating the required different mechanical properties of annular subsections.

BRIEF DESCRIPTION OF THE DRAWINGS

These and other aspects of the invention will be apparent from and elucidated with reference to the embodiment(s) described hereinafter. In the following drawings

Fig. 1 shows a system for pressure sensing employing the present pressure sensing device in a blood vessel in a schematic representation;
Fig. 2 shows a schematic representation of the cylindrical coordinate system used in the present invention;

Fig. 3 shows a schematic depiction of a part of a pressure sensing device according to the present invention;

Fig. 4a and 4b show a schematic depiction of how a non-radial symmetric coating pattern turn hydrostatic pressure into shape changes; and

Fig. 5 show a schematic depiction of a part of a pressure sensing device with different asymmetry of the coatings applied to the optical fiber.

## DETAILED DESCRIPTION OF THE INVENTION

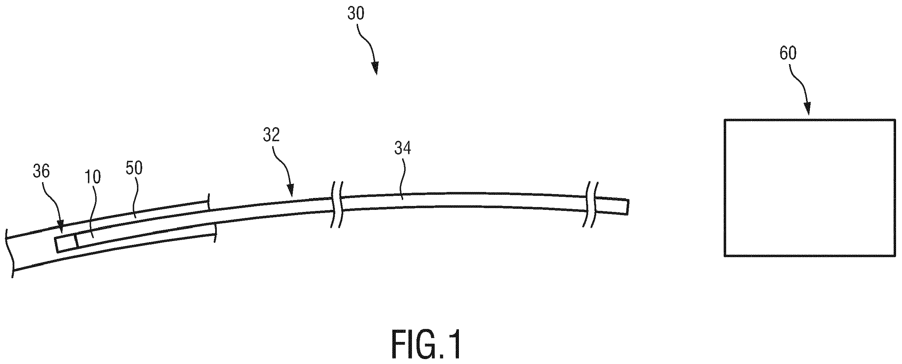

With reference to Fig. 1, a system for pressure sensing 30 will be described. The system 30 affords determining blood pressure in a blood vessel. The system comprises the present pressure sensing device 10 configured to be insertable into a blood vessel 50. The system 30 further comprises an interventional device 32. The interventional device 32 maybe a catheter, a guidewire or the like. At least a portion of the interventional device 32 is suitable for being introduced into a blood vessel 50 of a patient. The interventional device 32 comprises an elongated shaft 34 which may have a length of more than 1 m. The tip 36, such as an atraumatic tip, of the interventional device 32 is adapted for reciprocating movement in the blood vessel 50 without injuring the same.

The system 30 further comprises a workstation or console 60, to which the interventional device 32 maybe connected for communication, in particular optical communication of one or more console components. A part of length of the interventional device 32 are configured to be insertable into a blood vessel 22.

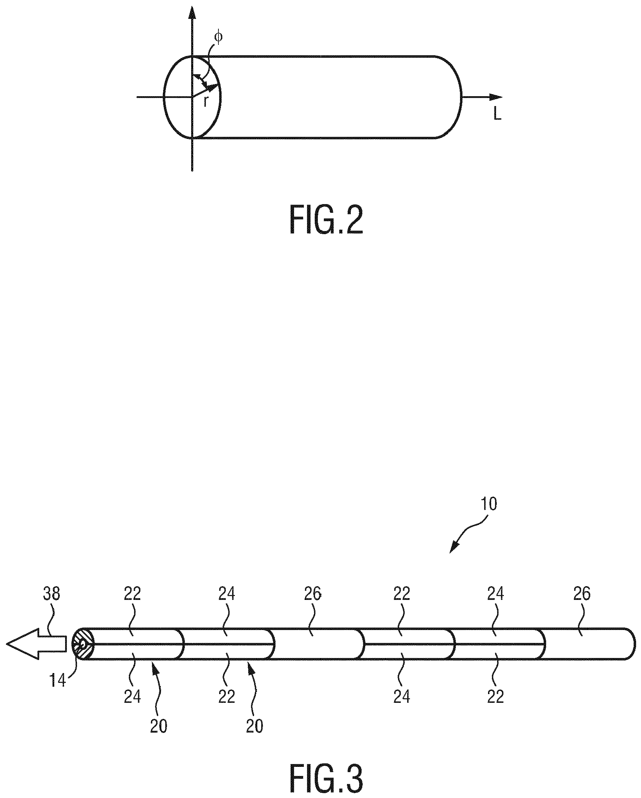

Fig. 2 shows a schematic representation of the cylindrical coordinate system used in the present invention. The axial dimension of the optical fiber is the length L, which goes from proximal to distal. Forces, stress, strain and other vectors that are (approximately) parallel to the length are called longitudinal in the present application. Forces and other vectors that are (approximately) perpendicular to the axis are called radial. Properties and forces can vary with the azimuth angle $\varphi$. Properties that are independent of the azimuth $\varphi$ are radial symmetric.

Fig. 3 shows a part of a pressure sensing device 10 according to the present invention. The pressure sensing device 10 comprises an optical fiber core 14 with at least one longer or multiple shorter FBGs written in. Alternatively, the fiber may contain multiple fiber cores with their own FBGs. If a single-core fiber is used, the core is preferably offset from the neutral axis. The fiber core 14 is coated with coatings that are in direct pressure contact with the blood stream. The coating is not axially symmetric. Rather, a plurality of longitudinal sections 20 along the length of the device each exhibit first annular subsection 22, i.e. portion, preferably one-half, of each longitudinal section 20 (for example the azimuthal angle $0° \leq \varphi < 180°$) coated with a first coating material and second annular subsection 24 (azimuthal angle $180° \leq \varphi < 360°$) coated with a second coating material. At other locations along the length of the device the coatings may be reversed or the fiber may be coated with just one axially symmetric coating 26. Preferably the accumulated length Lo of the device with the original order of both coatings is equal to the accumulated length $L_l$ of the device with the reversed coating. The first coating material and the second coating material have different values of the Poisson's number and/or the Young's modulus. Ideally, the first coating material is a kind of rubber, with a Poisson's number $v_1 \approx 0.49$ and the second coating material has Poisson's number $v_2$ of about 0.2 or below. Ideally the first coating and the second coating have similar thermal expansion coefficients $CTE_1$ and $CTE_2$, wherein a deviation of $CTE_1$ and $CTE_2$ is $\leq 10 \%$ referred to the lowest value of $CTE_1$ and $CTE_2$. Furthermore, the fiber core(s) 14 are connected to an optical interrogator (shown by reference numeral 38), preferably an FGB interrogator. The other side of fiber core(s) 14 extents towards or is an atraumatic tip.

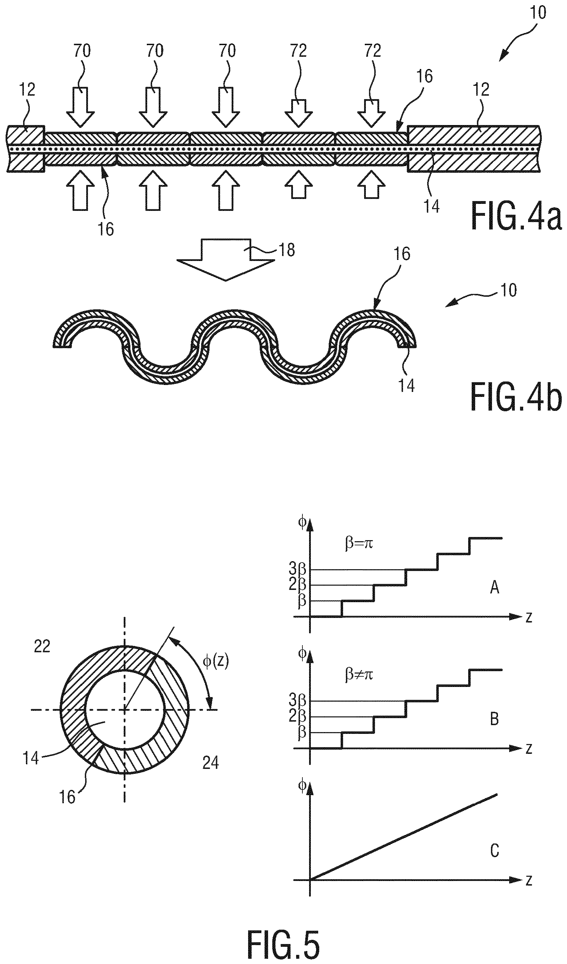

Fig. 4a and 4b show a schematic depiction of how a non-radial symmetric coating pattern turn hydrostatic pressure into shape changes. As may be derived from Fig. 4a, the hydrostatic pressure of the blood exerts a radial force (stress) 70, 72 on the coating 16. This force causes a compression (axial strain) of the coating in the radial direction coupled with an expansion (transversal stress) in the longitudinal direction. The ratio of stress (or pressure) to strain is given by the Young's modulus while the ratio of transverse strain to axial strain is given by the Poisson's number of the coating material. In general, the hydrostatic pressure on the device 10 inside the blood vessel is independent of the azimuthal angle $\varphi$, i.e. radially symmetric. If the coating is also radially symmetric the resulting expansion in the longitudinal direction will be radially symmetric as well and the device and the fiber will simply expand in length according to equation (2) above. This is the case in the areas covered by the axially symmetric coating 26. But in the first annular subsections 22 covered with the first coating material and the second annular subsections 24 covered with the second coating material, the coating is not radially symmetric. The first coating will deform different from the second coating. As a result the device 10 will locally bend or deform (indicated by reference numeral 18), which is shown in Fig. 4b. This local change in shape can be read out via an optical interrogator 38 and be used to reconstruct the hydrostatic pressure. This reconstruction can be computationally difficult because thermal effect and additional strain can influence the reading. But if the first coating and the second coating have a similar or even a corresponding thermal expansion coefficient, then the bending effect is independent of temperature. Compensating for strain can be done by using optical shape sensing algorithms. An exact value for the bending effect caused by hydrostatic pressure for the device described in this embodiment is best performed using numerical simulations as indicated above with respect to equations (3) to (5).

For a polymer coating $E_1 = E_2 = 3$ GPa, with $\nu_1 = 0.49$ and $\nu_2 = 0.2$ and a coating thickness h = 75 $\mu$m a curvature of

$$\frac{\kappa}{\Delta P} = 10^{-6} \ \mu\text{m/N} \text{ or } 1.3 \ 10^{-04} \frac{1}{\text{mmHg·m}} \text{ is obtained.}$$

Fig. 5 shows a schematic depiction of a part of a pressure sensing device with different asymmetry of the coatings applied to the optical fiber. This embodiment is similar to Fig. 3 but uses a different asymmetry of the coatings applied to the first annular subsection 22 and the second annular subsection 24 of the optical fiber core 14. According to Fig. 3 the two different coatings are applied in an alternating manner along the circumference of the fiber. In this way the azimuthal starting positions of the respective coating sections are a multiple of $\pi$, as illustrated in diagram A. As a result of the symmetry of the forces applied to the optical fiber core 14 this case the fiber will locally bend as illustrated in Figs. 4b and will show an in-plane oscillatory behavior. This symmetry can be destroyed by not simply alternating both coating materials, but also by applying subsequent coating sections at an azimuthal angle different than n (diagram B) As a consequence the coating materials start to spiral around the longitudinal axis. Due to the asymmetry of the forces now being applied to the FBG, the fiber shape will now oscillate in 3D-space, taking on the shape of a helix. Since the FBG is now allowed to expand in 3D space, instead of in a 2D plane, it is expected that the resulting strain and curvature as a function of applied pressure is larger as compared to case A, increasing the pressure sensitivity of the device. Diagram C illustrates the situation where the azimuthal starting position of the coating sections changes continuously as a function of longitudinal position along the fiber: both coating sections do not change position in a discrete manner, but rather spiral around the optical fiber core 14 in a continuous way.

The present invention may therefore provide a pressure sensing device for an intravascular device, like a guidewire or microcatheter, containing an optical fiber with one or more fiber Bragg gratings wherein

the mechanical properties (especially the Young's modulus or the Poisson ratio) of the device are not radially symmetric on all points along the length of the catheter but vary with the azimuthal angle $\varphi$ of the fiber as a function of axial position L; so that hydrostatic pressure will exert radial forces on the device and the FBGs which can be detected to determine local hydrostatic pressure;

especially where those radial forces will lead to a local radial deformation (bending) of the device and/or the included fiber that can be detected;

especially where the mechanical properties are balanced in such a way that only hydrostatic pressure will lead to such a deformation (bending) but not thermal effects or strain. This can be done e.g. by using materials with similar thermal expansion coefficients and similar Young's modulus but different Poisson ratio;

especially where optical shape sensing methods are used to detect the deformations;

especially where the radial asymmetries are limited to certain measurement points along the length of the device;

especially where the radial asymmetries consist of one set of material parameters over half the radius (e.g. 0° $\leq$ (p <180°) and a second set of material parameters over the second half (e.g. 180° $\leq \varphi$ <360°);

especially where the radial asymmetries are staggered in such a way that local deformations mostly cancel each other out and do not lead to a strong deformation of the whole device, especially if this is done by alternating asymmetries by 180°;

especially where the asymmetry in the material parameters is in the form of a coating of the optical fiber. The asymmetry can be induced by either (1) physically applying the coating to the fiber in an asymmetric way, or (2) treating a symmetrically applied coating by e.g. light irradiation in an asymmetric way;

where optical shape sensing technology is used to read-out information on local pressure and where both hydrostatic pressure and device position is shown to the user.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program maybe stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Pressure sensing device (10) comprising:

   an optical fiber (12) comprising a central axis (L) and at least one optical fiber core (14), the at least one optical fiber core (14) having one or more reflective FBG structures, and
   a coating (16) surrounding the optical fiber (12), the coating (16) having mechanical properties which are radially asymmetric along the central axis (L).

2. Pressure sensing device (10) of claim 1, wherein the pressure sensing device (10) is adapted to determine a local pressure, the local pressure exerting radial forces on the coating (16).

3. Pressure sensing device (10) of claim 1, wherein the mechanical properties are balanced such that thermal influences and/or strain influences are minimized so that any deformation of the optical fiber will be preliminarily caused by pressure.

4. Pressure sensing device (10) of claim 1, wherein the coating (16) has at least two longitudinal sections (20), each of the at least two longitudinal sections (20) having a first annular subsection (22) made of a first material and a second annular subsection (24) made of a second material being different from the first material, wherein the first material has a thermal expansion coefficient $CTE_1$, a Young's modulus of elasticity $E_1$, and a Poisson ratio $\nu_1$ and the second material has a thermal expansion coefficient $CTE_2$, a Young's modulus of elasticity $E_2$, and a Poisson ratio $\nu_2$, wherein at least one of a deviation of $CTE_1$ and $CTE_2$ is $\leq$ 10 % referred to the lowest value of $CTE_1$ and $CTE_2$, a deviation of $E_1$ and $E_2$ is $\leq$ 10 % referred to the lowest value of $E_1$ and $E_2$, and a deviation of $\nu_1$ and $\nu_2$ is $\geq$ 75 % referred to the lowest value of $\nu_1$ and $\nu_2$.

5. Pressure sensing device (10) of claim 1, wherein each of the at least two annular sections (20) has a first annular subsection (22) and a second annular subsection (24), the first annular subsection (22) having an annular azimuth $\varphi_1$, wherein $0° \leq \varphi_1 < 180°$, the second annular subsection (24) having an annular azimuth $\varphi_2$, wherein $\varphi_1 + \varphi_2 = 360°$.

6. Pressure sensing device (10) of claim 5, wherein first annular subsections (22) of the at least two longitudinal sections (20) are staggered around the optical fiber (12) such that the first annular subsections (22) of the at least two longitudinal sections (20) do not overlap.

7. Pressure sensing device (10) of claim 6, wherein each of the at least two longitudinal sections (20) encompasses at least one of the one or more reflective FBG structures.

8. Pressure sensing device (10) of claim 6, further comprising at least one axially symmetric coating (26) with $0° \leq \varphi < 360°$.

9. Pressure sensing device (10) of any of claim 6, wherein the first annular subsection (22) has $0° \leq \varphi < 180°$, and the second annular subsection (24) has $180° \leq \varphi < 360°$.

10. System for pressure sensing (30), comprising
    the pressure sensing device (10) of claim 1, and
    an interventional device (32), wherein one end of the optical fiber (12) is coupled to the interventional device (32).

11. System of claim 10, wherein the interventional device (32) is a guidewire or a catheter.

12. Method for determining a pressure, comprising
    optically determining bending of an optical fiber (12) comprising a central axis (L) and at least one optical fiber core (14), the at least one optical fiber core (14) having one or more reflective FBG structures, wherein a coating (16) surrounds the optical fiber (12), the coating (16) having mechanical properties which are radially asymmetric along the central axis (L), and
    calculating the pressure or a pressure difference from the bending of the coating (16).

13. Method of claim 12, wherein the pressure is a blood pressure in a blood vessel (50).

14. Method of claim 12 or 13, wherein calculating the pressure difference from the bending of the optical fiber (12) is performed calibration measurements and/or FEM simulations.

15. Computer program comprising program code means for causing a computer to carry out the steps of the method as claimed in claim 12 when said computer program is carried out on the system of claim 10.

FIG.1

FIG.2

FIG.3

FIG.4a

FIG.4b

FIG.5

Europäisches
Patentamt

European
Patent Office

Office européen
des brevets

## EUROPEAN SEARCH REPORT

Application Number

EP 18 15 5462

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/106887 A1 (OMNISENS SA [CH]) 23 July 2015 (2015-07-23) * abstract * * figures 6B, 7 * * paragraphs [0057], [0096] * ----- | 1-15 | INV. G01L11/02 |
| A | WO 00/33046 A1 (CIDRA CORP [US]) 8 June 2000 (2000-06-08) * abstract * * page 6, paragraph 21-23 * * page 8, paragraph 8-10 * ----- | 4 | |

TECHNICAL FIELDS SEARCHED (IPC)

G01L

The present search report has been drawn up for all claims

| Place of search | Date of completion of the search | Examiner |
|---|---|---|
| The Hague | 6 August 2018 | Kister, Clemens |

EPO FORM 1503 03.82 (P04C01)

## ANNEX TO THE EUROPEAN SEARCH REPORT
## ON EUROPEAN PATENT APPLICATION NO.

EP 18 15 5462

This annex lists the patent family members relating to the patent documents cited in the above-mentioned European search report.
The members are as contained in the European Patent Office EDP file on
The European Patent Office is in no way liable for these particulars which are merely given for the purpose of information.

06-08-2018

| Patent document cited in search report | | | Publication date | Patent family member(s) | | | Publication date |
|---|---|---|---|---|---|---|---|
| WO 2015106887 | A1 | | 23-07-2015 | EP<br>US<br>WO | 3094952<br>2016341612<br>2015106887 | A1<br>A1<br>A1 | 23-11-2016<br>24-11-2016<br>23-07-2015 |
| WO 0033046 | A1 | | 08-06-2000 | AU<br>CA<br>EP<br>NO<br>US<br>WO | 1842000<br>2353452<br>1137919<br>324819<br>6490931<br>0033046 | A<br>A1<br>A1<br>B1<br>B1<br>A1 | 19-06-2000<br>08-06-2000<br>04-10-2001<br>10-12-2007<br>10-12-2002<br>08-06-2000 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150141854 A

- US 5297437 A


**Non-patent literature cited in the description**

- **K. BHOWMIK et al.** Experimental Study and Analysis of Hydrostatic Pressure Sensitivity of Polymer Fibre Bragg Gratings. *J. Lightwave Tech.,* June 2015, vol. 33 (12), 2456-2462